**Europäisches Patentamt**

**European Patent Office**

**Office européen des brevets**

(19)

(11) Veröffentlichungsnummer: **0 025 892
B1**

(12) # EUROPÄISCHE PATENTSCHRIFT

(45) Veröffentlichungstag der Patentschrift:
**21.09.83**

(51) Int. Cl.³: **C 07 C 33/26**, C 07 C 29/03

(21) Anmeldenummer: **80105118.6**

(22) Anmeldetag: **28.08.80**

(54) **Verfahren zur Hydroxylierung von Styrol und Styrolderivaten.**

(30) Priorität: **19.09.79 DE 2937768**

(43) Veröffentlichungstag der Anmeldung:
**01.04.81 Patentblatt 81/13**

(45) Bekanntmachung des Hinweises auf die Patenterteilung:
**21.09.83 Patentblatt 83/38**

(84) Benannte Vertragsstaaten:
**AT BE CH FR GB IT LI NL SE**

(56) Entgegenhaltungen:
**GB-A-634 118
GB-A-1 119 612
US-A-2 555 927
US-A-2 794 055**

**H.L. Yale et al. J. Am Chem Soc. 1950, 72, p. 3716–3718**

**Rodd's Chemistry of Carbon Compounds, 2nd Ed. Vol. III Part E S. 72, 73, 78 Kap. 1 «Aralkanediols, aralkanetriols»**

(73) Patentinhaber: **Degussa Aktiengesellschaft, Weissfrauenstrasse 9, D-6000 Frankfurt am Main 1 (DE)**

(72) Erfinder: **Käbisch, Gerhard, Dr., Palmstrasse 1, D-7888 Rheinfelden (DE)**
Erfinder: **Malitius, Horst, Blümleacker 5, D-7888 Rheinfelden (DE)**
Erfinder: **Raupach, Siegfried, Langentalstrasse 24, D-7888 Rheinfelden (DE)**
Erfinder: **Trübe, Rudolf, Ernst-Reuter-Strasse 22, D-7888 Rheinfelden (DE)**
Erfinder: **Wittmann, Hans, Katzenbuckelweg 12, D-7888 Rheinfelden (DE)**

Verfahren zur Hydroxylierung von Styrol und Styrolderivaten

Die Erfindung betrifft ein Verfahren zur Hydroxylierung von Styrol, dessen im aromatischen Kern oder an der Vinylgruppe durch eine Methylgruppe substituierten Derivaten oder dessen an der Vinylgruppe durch eine Hydroxymethylgruppe substituierten Derivaten durch Umsetzung mit Ameisensäure mit einer Konzentration über 20 Gewichtsprozent und Wasserstoffperoxid mit einer Konzentration von weniger als 50 Gewichtsprozent und in einer Menge von weniger als 2 Mol pro Mol zu hydroxylierender Doppelbindung.

Unter «Hydroxylierung» ist zu verstehen, dass an die gegebenenfalls substituierte Vinyldoppelbindung zwei Hydroxylgruppen angelagert werden, also ein vicinales Diol gebildet wird.

Aus der GB-A-1 119 612 ist es bereits bekannt, alkenylsubstituierte aromatische Verbindungen durch Umsetzung mit Ameisensäure einer Konzentration über 20 Gewichtsprozent und Wasserstoffperoxid einer Konzentration von weniger als 50 Gewichtsprozent in einer Menge von weniger als 2 Mol pro Mol zu hydroxylierender Doppelbindung zu hydroxylieren. Konkret beschrieben ist die Hydroxylierung von Isoeugenolmethylether unter Verwendung von 11,74 Mol Ameisensäure pro Mol zu hydroxylierender Doppelbindung. Dabei entsteht primär ein Ameisensäureester, der anschliessend verseift werden muss.

Aus J. Am. Chem. Soc. 1950, 72, Seiten 3716 bis 3718, ist es ferner bereits bekannt, Zimtalkohol durch Umsetzung mit Ameisensäure einer Konzentration über 20 Gewichtsprozent und Wasserstoffperoxid einer Konzentration von weniger als 50 Gewichtsprozent in einer Menge von weniger als 2 Mol pro Mol zu hydroxylierender Doppelbindung zu Phenylglycerin zu hydroxylieren. Die Ameisensäure wird in einer Menge von 18,2 Mol pro Mol zu hydroxylierender Doppelbindung eingesetzt, und es entsteht primär wiederum ein Glykolmonoformiat, das anschliessend unter Erzielung einer geringen Ausbeute verseift werden muss.

Auch aus «Rodd's Chemistry of Carbon Compounds, 2nd Ed., Vol. III, Part E, Seiten 72 bis 78» ist die Herstellung von Aralkandiolen und Aralkantriolen durch Umsetzung der entsprechenden Aralkene mit Ameisensäure und Wasserstoffperoxid und anschliessende Verseifung der primär gebildeten Diolmonoester bereits bekannt.

In der US-A-2 555 927 wird ebenfalls erläutert, dass bei der Hydroxylierung von olefinisch ungesättigten Verbindungen mit Ameisensäure und Wasserstoffperoxid in hohem Masse eine Veresterung des gewünschten Produkts auftritt. Es wird deshalb ein neues Verfahren vorgeschlagen, bei dem anstelle der Ameisensäure niedrigsiedende Alkylformiate eingesetzt werden.

Schliesslich ist auch aus der GB-A-634 118 bereits die Hydroxylierung von olefinisch ungesättigten Verbindungen mittels Ameisensäure und Wasserstoffperoxid bekannt. Als geeignete Olefine werden zwar eine grosse Anzahl der verschiedenartigsten Verbindungen genannt, speziell beschrieben ist jedoch nur die Hydroxylierung von Allylalkohol zu Glycerin. Es wird mehrmals empfohlen, auf ein Mol Olefin etwa 1 Mol Ameisensäure zu verwenden. Aus den konkreten Beispielen ergibt sich aber, dass unter diesen Bedingungen eine untragbar lange Reaktionszeit erforderlich ist, die nur durch eine beträchtliche Erhöhung der Ameisensäuremenge bei im wesentlichen gleichem Umsatz und gleicher Ausbeute auf ein vernünftiges Mass vermindert werden kann.

Das erfindungsgemässe Verfahren ist nun dadurch gekennzeichnet, dass man das zu hydroxylierende Olefin bei einer Temperatur zwischen 40 und 80 °C mit weniger als 2 Mol Ameisensäure pro Mol zu hydroxylierender Doppelbindung und dem Wasserstoffperoxid umsetzt, Ameisensäure mit einer Konzentration zwischen 20 und 100 Gewichtsprozent einsetzt und die Konzentration des Wasserstoffperoxids in der wässrigen Phase des Reaktionsgemisches während der Gesamtdauer der Umsetzung unter 15 Gewichtsprozent hält.

Vorzugsweise wird die Umsetzung bei einer Temperatur zwischen 45 und 60 °C vorgenommen. Das Wasserstoffperoxid wird vorteilhafterweise in einer Menge von 1,1 bis 1,5 Mol pro Mol zu hydroxylierender Doppelbindung eingesetzt. Bevorzugt wird ein Wasserstoffperoxid mit einer Konzentration von 15 bis 40 Gewichtsprozent verwendet.

Durch das erfindungsgemässe Verfahren können beispielsweise Styrol in Phenylglykol, die verschiedenen Vinyltoluole in die entsprechenden Tolylglykole, α-Methylstyrol in α-Methylphenylglykol oder Zimtalkohol in Phenylglycerin umgewandelt werden. Die Umsetzungen verlaufen überraschenderweise trotz der verhältnismässig milden Reaktionsbedingungen sehr glatt und führen innerhalb tragbarer Reaktionszeiten zu hohen Ausbeuten an den gewünschten vicinalen Diolen.

Bei der praktischen Durchführung des erfindungsgemässen Verfahrens wird vorzugsweise die gesamte einzusetzende Ameisensäure vorgelegt. Auch das gesamte einzusetzende Wasserstoffperoxid kann vorgelegt werden. In diesem Falle wird dann das zu hydroxylierende Olefin langsam zudosiert. Normalerweise ist es jedoch zweckmässiger, einen Teil, beispielsweise etwa ein Drittel, der Gesamtmenge des zu hydroxylierenden Olefins zusammen mit der Gesamtmenge an Ameisensäure vorzulegen und das Wasserstoffperoxid und die Restmenge des zu hydroxylierenden Olefins langsam zuzudosieren. In jedem Falle wird das Reaktionsgemisch intensiv gerührt. Zur Verbesserung des Umsatzes empfiehlt sich eine angemessene Nachreaktionszeit, nachdem alle Reaktionsteilnehmer im Reaktionsgefäss vereinigt sind.

Nach beendeter Umsetzung liegen die gebildeten vicinalen Diole üblicherweise in der wässrigen Phase gelöst vor. Für manche Zwecke können derartige wässrige Lösungen direkt weiterverwendet werden. In den meisten Fällen wird sich aber nicht nur der Restgehalt an Wasserstoffperoxid störend auswirken, sondern es ist auch erwünscht, dass die gebildeten vicinalen Diole in reinerer Form isoliert werden. Dann kommen für die Aufarbeitung verschiedene Massnahmen in Betracht.

Falls das eingesetzte Olefin nicht vollständig umgesetzt wurde oder zu einem geringen Anteil während der Umsetzung polymerisierte, so dass sich aus dem Reaktionsgemisch eine zweite Phase abscheidet, wird zunächst eine Phasentrennung vorgenommen. Alternativ kann auch das nicht umgesetzte Olefin abdestilliert werden.

In vielen Fällen ist es dann ratsam, das in der wässrigen Phase noch enthaltene Wasserstoffperoxid weitgehend zu zersetzen. Die Zersetzung kann durch längeres Stehenlassen bei höherer Temperatur (Digestion) oder durch die Einwirkung eines geeigneten Katalysators (z.B. Überleiten über einen Platin-Festbett-Kontakt) oder durch eine Kombination dieser beiden Massnahmen bewirkt werden.

Nun wird zweckmässigerweise das Reaktionsgemisch, gegebenenfalls nach Neutralisation der enthaltenen Ameisensäure, durch Abdestillieren von Wasser eingedickt. Sofern die enthaltene Ameisensäure neutralisiert worden war, schliesst sich dann eine Extraktion mit einem geeigneten Lösungsmittel, beispielsweise Äthylacetat, an. Aus dem Extrakt wird das Lösungsmittel abdestilliert und der verbleibende Rückstand an rohem Diol gegebenenfalls durch Erwärmen unter vermindertem Druck von noch enthaltenem Wasser befreit. Alternativ kann auch auf die Neutralisation der im Reaktionsgemisch enthaltenen Ameisensäure verzichtet werden. Dann ist es zweckmässig, entweder die Eindickung nach Art einer Wasserdampfdestillation kontinuierlich vorzunehmen und so lange fortzuführen, bis das Destillat nur noch geringe Mengen an Ameisensäure enthält. Oder die Ameisensäureabtreibung wird diskontinuierlich derart vorgenommen, dass man den Rückstand nach dem Abtreiben der Hauptmenge an Ameisensäure nochmals mit Wasser versetzt, erneut eindickt und diese Operation mehrmals wiederholt. Der verbleibende Rückstand an rohem Diol kann dann wiederum durch Erwärmen unter vermindertem Druck von noch enthaltenen flüchtigen Anteilen befreit werden.

Die so erhaltenen rohen Diole liegen mit Reinheitsgraden von etwa 88 bis 96% vor. Sofern eine noch weitergehende Reinigung erforderlich ist, kann diese in üblicher Weise durch Umkristallisation oder durch fraktionierte Destillation unter vermindertem Druck bewirkt werden.

Das erfindungsgemässe Verfahren wird durch die nachfolgenden Beispiele näher erläutert. Prozentangaben bedeuten, sofern nichts anderes angegeben ist, in allen Fällen Gewichtsprozente.

Beispiel 1

In einem mit Rührer, Rückflusskühler, Tropftrichter und Thermometer ausgestatteten 2-l-Mehrhalskolben, der auf 50°C thermostatisiert ist, werden vorgelegt und intensiv gemischt: 590 g (2,6 Mol) Wasserstoffperoxid (15%) und 141 g (3,0 Mol) Ameisensäure (98%).

Zu dieser Mischung werden zudosiert 208 g (2,0 Mol) Styrol (stabilisiert mit 0,5 g Hydrochinon), und zwar die ersten 70 g innerhalb von 10 Minuten und der Rest im Verlaufe von 4 Stunden. Nach insgesamt 10 Stunden wird das Reaktionsgemisch zunächst abgekühlt und durch Filtration von 8 g Styrol (und Polystyrol) befreit. Der Umsatz betrug demnach 96,2%. Danach wird das Reaktionsgemisch einer Wasserdampfdestillation bei Normaldruck unterworfen, bis die Ameisensäurekonzentration im wässrigen Destillat von anfänglich 7,8% auf 0,8% abgefallen ist. Durch Erhitzen unter vermindertem Druck werden die restlichen Anteile an Wasser und Ameisensäure entfernt. Es hinterbleibt ein Rückstand von 271 g mit einem Phenylglykolgehalt von 89,5%.

Beispiel 2

In einer Rührapparatur gemäss Beispiel 1 werden vorgelegt: 104 g (1,0 Mol) Styrol (stabilisiert mit 0,5 g 2,6-Di-tert.-butyl-4-methyl-phenol) und 141 g (3,0 Mol) Ameisensäure (98%).

In diese gerührte Mischung werden im Verlaufe einer Stunde bei 50°C 255 g (1,5 Mol) Wasserstoffperoxid (20%) eindosiert. Anschliessend wird eine weitere Stunde bei 50°C gerührt. Danach werden im Verlaufe von 4 Stunden weitere 208 g (2,0 Mol) Styrol (stabilisiert wie oben) und 595 g (3,5 Mol) Wasserstoffperoxid (20%) gleichzeitig bei 55°C eindosiert und weitere 4 Stunden nachgerührt.

Aus dem Reaktionsgemisch werden 25 g Styrol abdestilliert. Der Umsatz betrug demnach etwa 92%. Das Reaktionsgemisch wird mit 300 g Natronlauge (40%) neutral gestellt und durch Abtoppen von 300 ml Wasser etwas eingedickt. Das nunmehr zweiphasige Gemisch wird durch Überleiten über einen Festbettkatalysator (0,1% Platin auf Berlsätteln) von Wasserstoffperoxid-Resten befreit und fünfmal hintereinander mit je 200 ml Äthylacetat extrahiert. Beim Einengen der vereinigten Äthylacetat-Extrakte hinterbleiben 342 g Rückstand, was einer Phenylglykol-Rohausbeute von 90% entspricht. Durch Umkristallisation oder Totaldestillation unter vermindertem Druck kann das rohe Phenylglykol leicht in ein Reinprodukt mit einem Schmelzpunkt von 66–67°C übergeführt werden.

Beispiel 3

Das Beispiel 2 wird mit dem Unterschied wiederholt, dass anstelle von insgesamt 312 g (3,0 Mol) Styrol nunmehr 354 g (3,0 Mol) Vinyltoluol (handelsübliches meta/para-Isomerengemisch) eingesetzt werden.

Nach beendeter Umsetzung werden 28 g Vinyltoluol zurückgewonnen. Der Umsatz betrug demnach etwa 92%.

Bei der Einengung der Äthylacetat-Extrakte fallen 411 g rohes Tolylglykol an, was einer Ausbeute von 98% entspricht. Durch Destillation bei 0,1 Torr werden 318 g reines Diol (Siedepunkt 123–126 °C) gewonnen.

Beispiel 4

In einer Rührapparatur gemäss Beispiel 1 werden vorgelegt:
118 g (1,0 Mol) α-Methylstyrol (2-Phenylpropen) und
141 g (3,0 Mol) Ameisensäure (98%).

In diese gerührte Mischung werden im Verlaufe von 5 Stunden eindosiert
236 g (2,0 Mol) α-Methylstyrol und
850 g (5,0 Mol) Wasserstoffperoxid (20%).

Nach einer Nachreaktionszeit von weiteren 2 Stunden werden aus dem Reaktionsgemisch 17 g α-Methylstyrol abdestilliert. Der Umsatz betrug demnach 95%. Das Reaktionsgemisch wird mit 285 g Natronlauge (40%) neutralisiert und durch Abtoppen von 300 ml Wasser eingeengt. Es hinterbleibt ein Gemisch, das sich in zwei Phasen trennt. Die organische Phase wird abgetrennt und portionenweise mit dem vorher abgetoppten Wasser gewaschen.

Die Waschwässer werden mit der wässrigen Phase vereinigt, katalytisch von restlichem Wasserstoffperoxid befreit und fünfmal hintereinander mit je 200 ml Äthylacetat extrahiert. Durch Einengen der vereinigten Äthylacetat-Extrakte werden 70 g Diol mit einem Reinheitsgrad von 96% gewonnen.

Die abgetrennte organische Phase wird einer Totaldestillation unter vermindertem Druck unterworfen. Dabei fallen folgende Fraktionen an:

Fraktion 1:  90 g Wasser
Fraktion 2:  73 g (identifiziert als Acetophenon)
Fraktion 3: 247 g Diol (99%ig; Siedepunkt bei 0,1 Torr 104–106 °C)
Rückstand: 38 g (nicht identifiziert).

Insgesamt wurden demnach an 2-Phenylpropandiol-1,2 erhalten
247 g mit einem Reinheitsgrad von 99% = 245 g (100%)
70 g mit einem Reinheitsgrad von 96% = 67 g (100%),
zusammen 312 g (100%), was einer Ausbeute von 72% entspricht.

## Patentansprüche

1. Verfahren zur Hydroxylierung von Styrol, dessen im aromatischen Kern oder an der Vinylgruppe durch eine Methylgruppe substituierten Derivaten oder dessen an der Vinylgruppe durch eine Hydroxymethylgruppe substituierten Derivaten durch Umsetzung mit Ameisensäure mit einer Konzentration über 20 Gewichtsprozent und Wasserstoffperoxid mit einer Konzentration von weniger als 50 Gewichtsprozent und in einer Menge von weniger als 2 Mol pro Mol zu hydroxylierender Doppelbindung, dadurch gekennzeichnet, dass man das zu hydroxylierende Olefin bei einer Temperatur zwischen 40 und 80 °C mit weniger als 2 Mol Ameisensäure pro Mol zu hydroxylierender Doppelbindung und dem Wasserstoffperoxid umsetzt, Ameisensäure mit einer Konzentration zwischen 20 und 100 Gewichtsprozent einsetzt, und die Konzentration des Wasserstoffperoxids in der wässrigen Phase des Reaktionsgemisches während der Gesamtdauer der Umsetzung unter 15 Gewichtsprozent hält.

2. Verfahren nach Anspruch 1, dadurch gekennzeichnet, dass man die Umsetzung bei einer Temperatur zwischen 45 und 60 °C vornimmt.

## Claims

1. A process for the hydroxylation of styrene, the derivatives thereof which are substituted by a methyl group in the aromatic nucleus or at the vinyl group, or the derivatives thereof which are substituted by a hydroxymethyl group at the vinyl group, by a reaction with formic acid having a concentration of more than 20% by weight, and hydrogen peroxide having a concentration of less than 50% by weight and in a quantity of less than 2 mols per mol of double bond to be hydroxylated, characterised in that the olefin to be hydroxylated is reacted with less than 2 mols of formic acid per mol of double bond to be hydroxylated and with the hydrogen peroxide at a temperature of from 40 to 80 °C, formic acid having a concentration of from 20 to 100% by weight is used, and the concentration of the hydrogen peroxide in the aqueous phase of the reaction mixture is maintained below 15% by weight for the complete duration of the reaction.

2. A process according to claim 1, characterised in that the reaction is carried out at a temperature of from 45 to 60 °C.

## Revendications

1. Procédé d'hydroxylation du styrène, de ses dérivés substitués par un groupe méthyle sur le noyau aromatique ou sur le groupe vinyle, ou de ses dérivés substitués par un groupe hydroxyméthyle sur le groupe vinyle, par réaction avec de l'acide formique à une concentration supérieure à 20% en poids et de l'eau oxygénée à une concentration inférieure à 50% en poids et en une proportion inférieure à 2 moles par mole de double liaison à hydroxyler, procédé caractérisé en ce que l'on fait réagir l'oléfine à hydroxyler à une température comprise entre 40 et 80 °C, avec moins de 2 moles d'acide formique par mole de double liaison à hydroxyler et avec de l'eau oxygénée, que l'on met en œuvre de l'acide formique à une concentration comprise entre 20 et 100% en poids, et que l'on maintient la concentration de l'eau oxygénée dans la phase aqueuse du mélange réactionnel, pendant toute la durée de la réaction, en-dessous de 15% en poids.

2. Procédé selon la revendication 1, caractérisé en ce que l'on effectue la réaction à une température comprise entre 45 et 60 °C.